# EUROPÄISCHE PATENTANMELDUNG

(11) **EP 0 535 647 A2**
(43) Veröffentlichungstag der Anmeldung: **07.04.1993**
(21) Anmeldenummer: 92116767.2
(22) Anmeldetag: 30.09.1992
(51) Int. Cl.: A61F 13/15

(54) **Windelhöschen für Babys und Kleinkinder**

(30) Priorität: 01.10.1991 DE 9112256 U
(71) Anmelder: Fretchies Baby- und Kinderartikel GmbH, D-65936 Frankfurt (DE)
(72) Erfinder: Dehnert, Heinz, W-6000 Frankfurt am Main (DE); Dehnert, Christine, W-6000 Frankfurt am Main (DE)
(74) Vertreter: Popp, Eugen, Dr.

(57) **Zusammenfassung**

Windelhöschen für Babys und Kleinkinder mit einem der Körperanatomie des Babys bzw. Kleinkindes angepaßten Außenteil (12), einem saugfähigen, als Einlage in das Außenteil (12) vorgesehenen Innenteil (14) aus kochfestem Baumwollmaterial, Zellstoffmaterial oder dgl. sowie einer Feuchtigkeitssperre (30), wobei die Feuchtigkeitssperre (30) vollständig in das Außenteil (12) und/oder in das Innenteil (14) eingebettet ist und wobei das Außenteil (12) aus kochfestem Baumwollmaterial, Zellstoffmaterial oder dgl. besteht und die Feuchtigkeitssperre (30) aus kochfester Kunststoffolie oder dgl. gebildet ist.

## Beschreibung

Die Erfindung betrifft ein Windelhöschen für Babys und Kleinkinder mit einem der Körperanatomie des Babys bzw. Kleinkindes angepaßten Außenteil, einem saugfähigen, als Einlage in das Außenteil vorgesehenen Innenteil aus kochfestem Baumwollmaterial, Zellstoffmaterial oder dgl. sowie einer Feuchtigkeitssperre.

Derartige Windelhöschen für Babys und Kleinkinder sind allgemein bekannt. So ist in dem DE 91 02 591.5 U1 ein Windelhöschen beschrieben, das über ein eingearbeitetes natürliches Saugpolster als Innenteil aus vorzugsweise Baumwollmaterial verfügt, dessen Saugkraft durch Kochwäsche regenerierbar ist, und das auf der ganzen Fläche nach außen eine Feuchtigkeitssperre umfaßt. Durch Verwendung von Baumwollmaterial sollen Hautreizungen und Allergien des Babys bzw. Kleinkindes vermindert werden, da Baumwollmaterial in der Regel weich und anschmiegsam ist, eine hohe Saugkraft zur Aufnahme von Feuchtigkeit aufweist und infolge Kochfestigkeit hygienisch ist. Durch die Feuchtigkeitssperre soll darüber hinaus vermieden werden, daß die von dem Baumwollmaterial aufgenommene Feuchtigkeit mit das Windelhöschen umhüllenden Kleidungsstücken in Verbindung gelangt.

Das bekannte Windelhöschen, das im Handel erhältlich ist, weist allerdings einige Nachteile auf. Das Innenteil ist auf das Außenteil, nämlich auf eine als Feuchtigkeitssperre vorgesehene Gummischicht, welche mit einem äußeren Überzug aus Baumwollmaterial oder dgl. versehen ist, aufgenäht. Da einerseits die Nahtverbindungen zwischen dem Innenteil und der Gummischicht des Außenteiles zur Haut des Babys bzw. Kleinkindes weisen und andererseits die als Feuchtigkeitssperre vorgesehene Gummischicht des Außenteiles aufgrund ungenügender Abdeckung durch das Innenteil an der Haut des Babys bzw. Kleinkindes anliegt, kommt es durch das bekannte Windelhöschen trotz Verwendung eines aus Baumwollmaterial oder dgl. bestehenden Innenteiles häufig zu Hautreizungen oder sogar Allergien des Babys bzw. Kleinkindes. Da die als Feuchtigkeitssperre vorgesehene Gummischicht weiterhin ausgesprochen steif ausgebildet und zudem verhältnismäßig schwer ist, weist das bekannte Windelhöschen eine nur sehr geringe Bequemlichkeit für das Baby bzw. Kleinkind auf. Da die als Feuchtigkeitssperre vorgesehene Gummischicht, auf welche das aus kochfestem Baumwollmaterial oder dgl. bestehende Innenteil aufgenäht ist, keine hohe Temperaturbeständigkeit aufweist, läßt sich das bekannte Windelhöschen zu Lasten der Hygiene nur insgesamt waschen und deswegen nur bedingt der Kochwäsche unterziehen.

Des weiteren ist der DE-PS 905 123 ein Windelhöschen aus Baumwolle, Zellwolle oder ähnlichen Stoffen entnehmbar, das teilweise mit einer wasser-, säure- und kochfesten Kunststoffolie ausgekleidet ist. Die Kunststoffolie ist innenseitig auf das Windelhöschen aufgenäht, aufgeklebt, aufgeknöpft oder dgl. Vor dem Wickeln des Babys bzw. Kleinkindes wird auf die Kunststoffolie eine Packung aus saugfähigem Material, wie z. B. Zellstoff oder dgl., aufgelegt und mittels am Rand der Packung vorgesehener Schlaufen an der mit Häkchen versehenen Kunststoffolie festgelegt. Die Handhabung dieses bekannten Windelhöschens beim Wickeln des Babys bzw. Kleinkindes ist infolgedessen verhältnismäßig aufwendig.

Schließlich ist im Handel noch ein Windelhöschen erhältlich, das ein der Körperanatomie des Babys bzw. Kleinkindes angepaßtes Außenteil und ein in das Außenteil eingenähtes, als Polsterung vorgesehenes Innenteil umfaßt, wobei das Außenteil wie auch das Innenteil aus einem Baumwollmaterial oder dgl. bestehen. Als Feuchtigkeitssperre ist ein Nylonhöschen vorgesehen, welches zusätzlich über das bekannte Windelhöschen, das zuvor angelegt ist, übergezogen werden muß. Die Handhabung des bekannten Windelhöschens beim Wickeln des Babys bzw. Kleinkindes ist daher ebenfalls verhältnismäßig aufwendig.

Der Erfindung liegt daher die Aufgabe zugrunde, die vorgenannten Probleme zu vermeiden, d. h. ein Windelhöschen für Babys und Kleinkinder der eingangs genannten Art zu schaffen, das Feuchtigkeit sicher aufnimmt und hält, Hautreizungen und Allergien bei den Babys bzw. Kleinkindern vermeidet sowie bequem, kochfest und leicht handhabbar ist.

Diese Aufgabe wird dadurch gelöst, daß die Feuchtigkeitssperre vollständig in das Außenteil und/oder in das Innenteil eingebettet ist, wobei das Außenteil aus kochfestem Baumwollmaterial, Zellstoffmaterial oder dgl. besteht und die Feuchtigkeitssperre aus kochfester Kunststoffolie oder dgl. gebildet ist.

Auf diese Weise läßt sich ein Windelhöschen für Babys und Kleinkinder erhalten, das Feuchtigkeit durch das Innenteil und ggf. noch zusätzlich durch das Außenteil, welche jeweils aus einem saugfähigen Baumwollmaterial, Zellstoffmaterial oder dgl. bestehen, sicher aufsaugt, die von der vollständig in das Außenteil und/oder in das Innenteil eingebetteten Feuchtigkeitssperre, welche aus kochfester Kunststoffolie oder dgl. gebildet ist, gehalten wird. Somit ist sichergestellt, daß die Feuchtigkeit aus dem erfindungsgemäßen Windelhöschen nicht austreten und mit der das Windelhöschen umgebenden Kleidung des Babys bzw. Kleinkindes in Berührung kommen kann. Weiterhin ruft das erfindungsgemäße Windelhöschen aufgrund der vollständigen Einbettung der Feuchtigkeitssperre in das Außenteil und/oder in das Innenteil bei den Babys bzw. Kleinkindern keine Hautreizungen oder sogar Allergien hervor, da die Kunststoffolie oder dgl. in jedem Fall von hautverträglichem Baumwollmaterial, Zellstoffmaterial oder dgl. umgeben bzw. abgedeckt ist. Insofern ist ein Kontakt zwischen der Kunststoffolie oder dgl. und der Haut der Babys bzw. Kleinkinder von vornherein ausgeschlossen. Weiterhin ist das erfindungsgemäße Windelhöschen wegen des besonders hautverträglichen, zudem ausschließlich mit der Haut der Babys bzw. Kleinkinder in Berührung kommenden weichen und anschmiegsamen Baumwollmaterials, Zellstoffmaterials oder dgl. des Außenteiles sowie des Innenteiles sowie wegen der im Außenteil bzw. Innenteil angeordneten, als Feuchtigkeitssperre vorgesehenen biegsamen sowie leichten Kunststoffolie äußerst bequem. Des weiteren ist das erfindungsgemäße Windelhöschen insgesamt kochfest, da das Baumwollmaterial, Zellstoffmaterial oder dgl. wie auch die Kunststoffolie oder dgl. von Außenteil und Innenteil der Kochwäsche unterzogen werden können. Schließlich läßt sich das erfindungsgemäße Windelhöschen ausgesprochen einfach handhaben.

Besonders vorteilhaft sind darüber hinaus die erfindungsgemäßen Maßnahmen nach Anspruch 2 und 3. Durch die Ausbildung der Feuchtigkeitssperre sowie deren Anordnung zu bzw. Verbindung mit dem jeweiligen Außenteil und/oder Innenteil läßt sich nämlich die von dem Baumwollmaterial, Zellstoffmaterial oder dgl. aufgesogene Feuchtigkeit noch besser in dem erfindungsgemäßen Windelhöschen halten. Auch wird die Bequemlichkeit noch zusätzlich dadurch erhöht, daß die Feuchtigkeitssperre mit dem Baumwollmaterial, Zellstoffmaterial oder dgl. des Außenteiles und/oder des Innenteiles vernäht ist, wobei die Verbindungsstellen in den Randbereich von Außenteil und Innenteil und somit aus dem Schrittbereich der Babys bzw. Kleinkinder heraus verlegt sind.

Durch die erfindungsgemäßen Merkmale nach den Ansprüchen 4 bis 7 ist es darüber hinaus möglich, die Saugkraft des Windelhöschens noch zusätzlich zu erhöhen bzw. in sonstiger Weise beliebig zu variieren.

Weiterhin sind die Maßnahmen nach Anspruch 8 für eine sichere Feuchtigkeitssperre von Bedeutung.

Die Merkmale nach den Ansprüchen 9 und 10 dienen einer vielseitigen Verwendungsmöglichkeit des erfindungsgemäßen Windelhöschens, da das Außenteil und das Innenteil separat voneinander gewaschen werden können, entsprechend beispielsweise dem Grad ihrer Verschmutzung. Auch ist es auf diese Weise möglich, den Babys bzw. Kleinkindern ausschließlich das Außenteil ohne das Innenteil des Windelhöschens anzulegen.

Durch die erfindungsgemäße Ausbildung des Außenteiles entsprechend den Merkmalen nach den Anspruch 11 ist gewährleistet, daß das Windelhöschen an die jeweilige Körperanatomie des Babys bzw. Kleinkindes, d. h. insbesondere an dessen Größe, angepaßt werden kann.

Desweiteren liegt es im Rahmen der Erfindung, den Klettverschluß von Außenteil und/oder Innenteil entsprechend den Maßnahmen nach Anspruch 12 mit integriertem Fusselschutz zu versehen. Auf diese Weise ist sicher ausgeschlossen, daß der Klettverschluß während des eigentlichen Wasch- bzw. Kochvorganges mit Fusseln, Fädchen oder dgl. anderer Kleidungsstücke oder ähnlichem zugesetzt und auf diese Weise die Funktions-, d. h. Haftfähigkeit des Klettverschlusses erheblich gemindert wird.

Schließlich ist es noch von Vorteil, daß das Außenteil gemäß den Merkmalen nach Anspruch 13 jeweils randseitig wenigstens im Bereich der Beine des Babys bzw. Kleinkindes mit einem Gummiband oder dgl. versehen ist. Das Windelhöschen weist somit eine ausreichende Dichtigkeit zum Zurückhalten aufgetretener Feuchtigkeit im zumeist unten liegenden Bereich der Beine auf.

Weitere Merkmale, Vorteile und Einzelheiten der Erfindung ergeben sich aus der nachfolgenden Beschreibung einer bevorzugten Ausführungsform der Erfindung sowie anhand der Zeichnung. Hierbei zeigen
- Fig. 1: eine perspektivische Vorderansicht auf eine Ausführungsform eines erfindungsgemäß ausgebildeten Windelhöschens;
- Fig. 2: eine Draufsicht auf das Außenteil des erfindungsgemäßen Windelhöschens nach Fig. 1;
- Fig. 3: eine Seitenansicht auf das Außenteil des erfindungsgemäßen Windelhöschens nach Fig. 2;
- Fig. 4: eine Draufsicht auf das Innenteil des erfindungsgemäßen Windelhöschens nach Fig. 1;
- Fig. 5: eine Seitenansicht auf das Innenteil des erfindungsgemäßen Windelhöschens nach Fig. 4;
- Fig. 6: einen Querschnitt durch eine Ausführungsform des Außenteiles von dem erfindungsgemäßen Windelhöschen gemäß Linie VI-VI in Fig. 2 in vergrößerter Darstellung;
- Fig. 7: einen Querschnitt durch eine Ausführungsform des Innenteiles von dem erfindungsgemäßen Windelhöschen gemäß Linie VII-VII in Fig. 4 in vergrößerter Darstellung;
- Fig. 8: einen Querschnitt durch eine andere Ausführungsform des Außenteiles von dem erfindungsgemäßen Windelhöschen entsprechend Fig. 6;
- Fig. 9: einen Querschnitt durch eine andere Ausführungsform des Innenteiles von dem erfindungsgemäßen Windelhöschen entsprechend Fig. 7., und
- Fig. 10: einen Querschnitt durch eine weitere Ausführungsform des Innenteiles von dem erfindungsgemäßen Windelhöschen entsprechend Fig. 7.

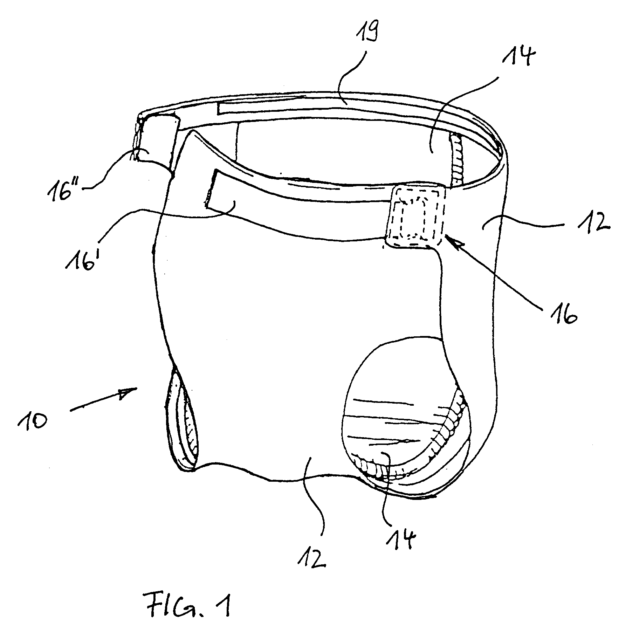

Das in Fig. 1 dargestellte Windelhöschen 10 für Babys und Kleinkinder (nicht dargestellt) umfaßt ein Außenteil 12, das an die Körperanatomie des Babys bzw. Kleinkindes angepaßt ist, sowie ein saugfähiges Innenteil 14, das als Einlage in das Außenteil 12 vorgesehen ist und aus kochfestem Baumwollmaterial, Zellstoffmaterial oder dgl. besteht.

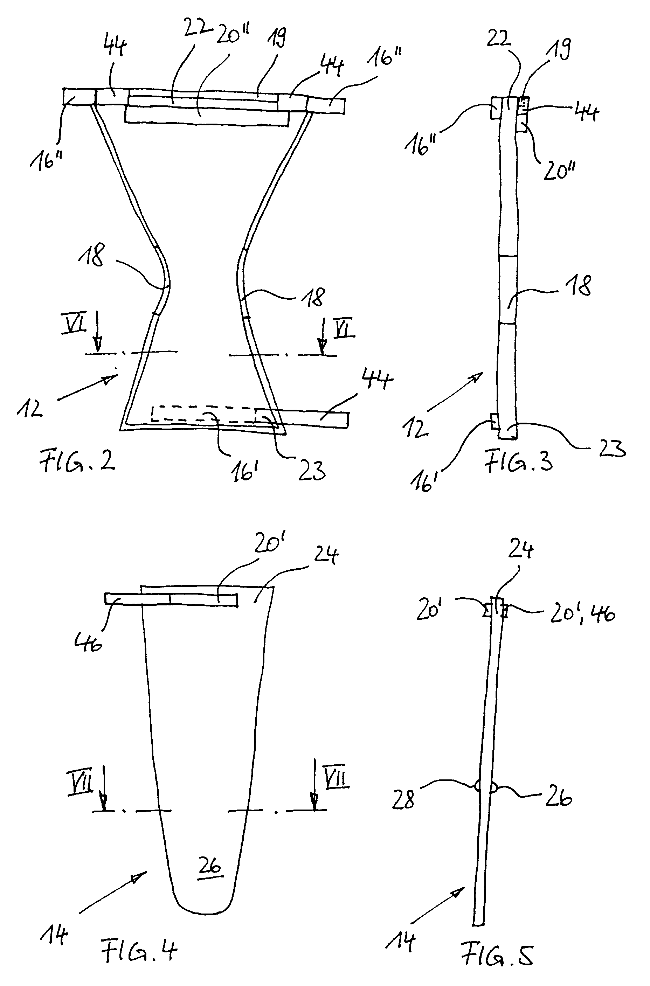

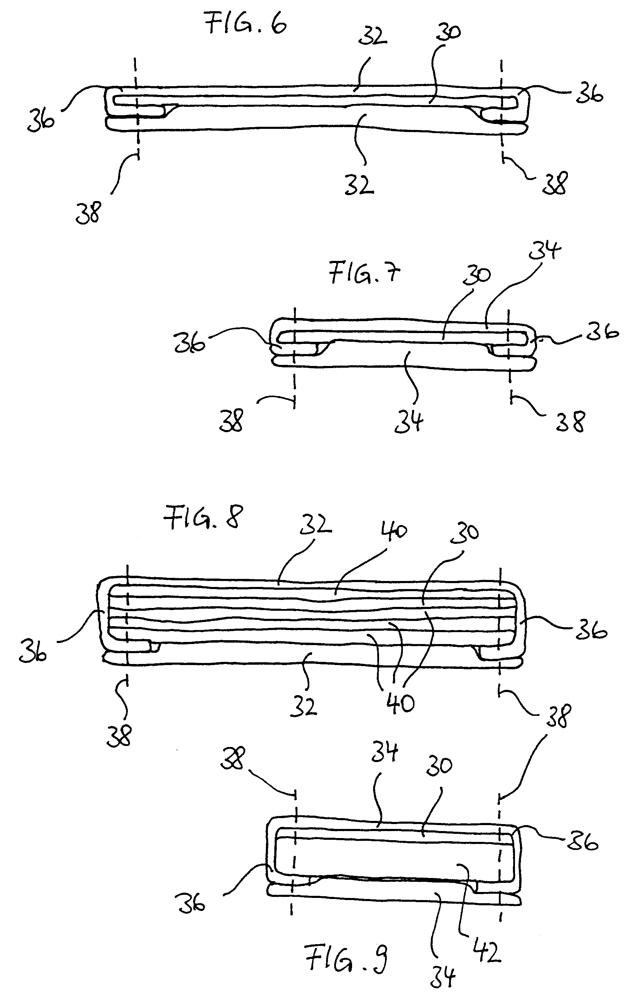

Entsprechend den Fig. 2 und 3 weist das Außenteil 12 einen Klettverschluß 16 mit beispielsweise schlaufenförmigen Elementen 16' und hakenförmigen Elementen 16'' auf, mittels welchem das Außenteil 12 gemäß Fig. 1 verschließbar ist. Der Klettverschluß 16 läßt sich dabei aufgrund seiner Weltenverstellbarkeit genau an den Körper des Babys bzw. Kleinkindes - entsprechend dessen jeweiliger Größe - anpassen, so daß das Windelhöschen 10 mit nur wenigen Handgriffen angelegt ist und bequem wie auch zuverlässig am Körper des Babys bzw. Kleinkindes sitzt. Zur Weitenverstellbarkeit sind hier beispielsweise die schlaufenförmigen Elemente 16' des Klettverschlusses 16 länglich entland der Endseite 23 verlaufend ausgebildet.

Weiterhin ist das Außenteil 12 jeweils randseitig wenigstens im Bereich der nicht dargestellten Beine des Babys bzw. Kleinkindes mit einem Gummiband 18 oder dgl. versehen. Hierdurch kommt das Außenteil 12 auf der Haut des Babys bzw. Kleinkindes im Bereich von dessen Beinen unmittelbar zur Anlage, so daß aufgetretene und von dem Innenteil 14 aufgesaugte Feuchtigkeit in dem Windelhöschen 10 sicher zurückgehalten wird und nicht an dem Außenteil 12 vorbei nach außen austreten kann. Das Außenteil 12 besteht ebenfalls wie das Innenteil 14 aus kochfestem Baumwollmaterial, Zellstoffmaterial oder dgl. Um darüber hinaus einen zusätzlichen Halt des Windelhöschens 10 zu erhalten, ist im Bereich einer der beiden Endseiten 22, 23 des Außenteiles 12 ein Gummiband 19 oder dgl. eingezogen.

Wie weiterhin in den Fig. 2 und 3 angedeutet, kann der Klettverschluß 16 des Außenteiles 12, d. h. hier beispielsweise die hakenförmigen Elemente 16'' bzw. schlaufenförmige Elemente 16' durch einen an dem Außenteil 12 integrierten Fusselschutz 44 in Form einer Schutzabdeckung oder dgl.versehen sein. Als Fusselschutz 44 können beispielsweise schlaufenförmige Elemente entsprechend derjenigen
Elemente 16' bzw. hakenförmige Elemente entsprechend derjenigen Elemente 16'' vorgesehen sein, um die hakenförmigen Elemente 16'' bzw. schlaufenförmigen Elemente 16' abzudecken und somit gegen Fusseln, Fädchen vor allem beim eigentlichen Wasch- bzw. Kochvorgang zu schützen. Zu diesem Zweck sind dann die hakenförmigen Elemente 16'', welche nach Fig. 2 nach außen, d. h. nach rechts und nach links von dem Außenteil 12 wegstehen, um 180° nach innen, zueinander gewandt umzuklappen bzw. die nach rechts wegstehende Lasche in Fig. 2 nach links um 180° nach innen umzulegen.

Das in den Fig. 4 und 5 dargestellte, etwa zungenförmig ausgestaltete Innenteil 14 ist gemäß Fig. 1 in das Außenteil 12 eingelegt und mit dem Außenteil 12 über eine lösbare Befestigungsvorrichtung, insbesondere einen Klettverschluß 20, einen Druckknopf-, Rast-, Hak- oder dgl. -verschluß verbunden. Der Klettverschluß 20 umfaßt beispielsweise zum einen schlaufenförmige Elemente 20', welche an dem Innenteil 14 angeordnet sind, und zum anderen hakenförmige Elemente 20'', welche an dem Außenteil 12 angeordnet sind.

Um Verwerfungen oder dgl. des Innenteiles 14 in dem Außenteil 12 zu vermeiden, ist der das Innenteil 14 mit dem Außenteil 12 verbindende Klettverschluß 20 im Bereich einer der einander zugeordneten Endseiten 22, 24 von Innenteil 12 und Außenteil 12 angebracht. Demnach sind die schlaufenförmigen Elemente 20' des Klettverschlusses 20 an der Endseite 24 des Innenteiles 14 und die hakenförmigen Elemente 20'' des Klettverschlusses 20 an der Endseite 22 des Außenteiles 12 angeordnet. Um die Handhabbarkeit des Windelhöschens 10 während des Anlegens noch weiter zu vereinfachen, sind die schlaufenförmigen Elemente 20' des Klettverschlusses 20 sowohl auf der Oberseite 26 als auch auf der Unterseite 28 im Bereich der Endseite 24 des Innenteiles 14 angeordnet.

Des weiteren ist der Klettverschluß 20 des Innenteiles 14 entsprechend Fig. 4 mit einem integrierten Fusselschutz 46 in Form einer Schutzdeckung versehen. Dieser besteht beispielsweise aus einer Lasche mit hier hakenförmigen Elementen, entsprechend derjenigen Elemente 20'', welche die schlaufenförmigen Elemente 20' des Klettverschlusses 20 durch einfaches Umklappen um 180° für den eigentlichen Wasch- bzw. Kochvorgang abdeckt und somit gegenüber Fusseln, Fädchen oder dgl. schützt. Der Fusselschutz 46, der in Fig. 4 außer Funktion ist, ist daher dann deckungsgleich mit den schlaufenförmigen Elementen 20'.

Ungeachtet dessen kann der Fusselschutz 44 auch aus hakenförmigen Elementen 16'' und der Fusselschutz 46 aus schlaufenförmigen Elementen 20' gebildet sein, ohne daß hierdurch die Schutzfunktion negativ beeinträchtigt würde.

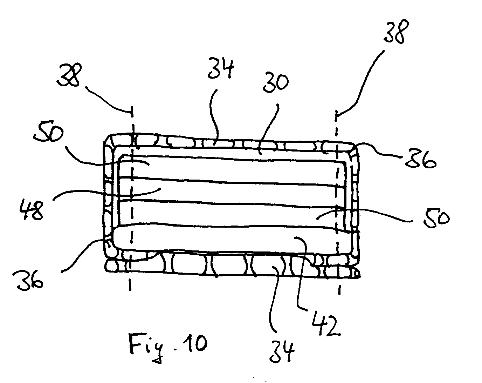

Wie in den Fig. 6 bis 10 dargestellt, ist eine Feuchtigkeitssperre 30 vollständig in das Außenteil 12 und in das Innenteil 14 eingebettet. Die Feuchtigkeitssperre 30 ist dabei aus Kunststoffolie oder dgl. gebildet, die sowohl kochfest als auch säurebeständig ist. Die als Feuchtigkeitssperre 30 vorgesehene Kunststoffolie oder dgl. ist entsprechend den Fig. 6 und 7 wenigstens einlagig zwischen zwei Lagen 32 bzw. 34 aus Baumwollmaterial, Zellstoffmaterial oder dgl. des Außenteiles 12 bzw. des Innenteiles 14 angeordnet. Die Feuchtigkeitssperre 30, d.h. die eine Lage aus Kunststoffolie oder dgl., sowie die beiden Lagen 32 bzw. 34 aus Baumwollmaterial, Zellstoffmaterial oder dgl. des Außenteiles 12 bzw. des Innenteiles 14 sind zueinander deckungsgleich und miteinander unlösbar, beispielsweise durch Vernähen, verbunden. Die gegenseitige Verbindung ist dabei vorzugsweise im randseitigen Bereich 36 von Außenteil 12 bzw. Innenteil 14, wie durch die Strichlinien 38 angedeutet, erfolgt.

Die Figuren 8 und 9 zeigen zwei weitere Ausführungsformen von Außenteil 12 und Innenteil 14 des Windelhöschens 10. Entsprechend Fig. 8 sind neben zwei Lagen der Feuchtigkeitssperre 30 aus Kunststoffolie oder dgl. weitere Lagen 40 aus Baumwollmaterial, Zellstoffmaterial oder dgl. zwischen den beiden Lagen 32 aus Baumwollmaterial, Zellstoffmaterial oder dgl. bestehend des Außenteiles 12 eingebracht. Sämtliche Lagen 30, 32, 40 aus Kunststoffolie und Baumwollmaterial, Zellstoffmaterial oder dgl. sind miteinander im randseitigen Bereich 36 entsprechend der Strichlinien 38 unlösbar verbunden.

Nach Fig. 9 ist neben der Feuchtigkeitssperre 30 aus Kunststofffolie oder dgl. eine dicke Lage 42 aus Vliesstoff oder dgl. zwischen den beiden Lagen 34 aus Baumwollmaterial, Zellstoffmaterial oder dgl. des Innenteiles 14 eingebracht. Auch die Lagen 30, 34 und 42 aus Kunststoffolie, Baumwollmaterial, Zellstoffmaterial sowie Vliesstoff oder dgl. sind sämtlich miteinander im randseitigen Bereich 36 verbunden.

In Fig. 10 ist schließlich noch eine andere Ausführungsform eines Innenteiles 14 des Windelhöschens 10 dargestellt. So bestehen die beiden Lagen 34 hier vorzugsweise aus grobmaschigem Baumwollnetz oder dgl., wodurch ein angenehmes Traggefühl dem Baby bzw. Kleinkind vermittelt wird. Zudem ist ein solches grobmaschiges Baumwollnetz ausgesprochen hautverträglich. Darüberhinaus ist zwischen den beiden Lagen 34 aus grobmaschigem Baumwollnetz oder dgl. des Innenteiles 14 eine Lage 48 aus Watte und zwei Lagen 50 aus Molton oder dgl. eingebracht und von einer Lage der Feuchtigkeitssperre 30 aus Kunststoffolie und einer weiteren Lage 42 aus Vliesstoff vorgesehen. Sämtliche vorgenannten Lagen 30, 42, 48 und 50 sind dabei von den beiden Lagen 34 aus Baumwollmaterial , d. h. aus grobmaschigem Baumwollnetz, eingefaßt und mit diesem im randseitigen Bereich 36 entsprechend der Strichlinien 38 unlösbar verbunden.

Die vorliegende Erfindung ist nicht auf die beschriebenen Ausführungsbeispiele beschränkt. So ist es ebenso denkbar, daß das Außenteil 14 bzw. Innenteil 12 des Windelhöschens 10 in identischer oder abgewandelter Weise entsprechend dem Innenteil 14 gemäß Fig. 9 bzw. dem Außenteil 12 entsprechend Fig. 8 aufgebaut sind.

Sämtliche in den Anmeldungsunterlagen offenbarten Merkmale werden als erfindungswesentlich beansprucht, soweit sie einzeln oder in Kombination gegenüber dem Stand der Technik neu sind.

## Patentansprüche

1. Windelhöschen für Babys und Kleinkinder mit einem der Körperanatomie des Babys bzw. Kleinkindes angepaßten Außenteil (12), einem saugfähigen, als Einlage in das Außenteil (12) vorgesehenen Innenteil (14) aus kochfestem Baumwollmaterial, Zellstoffmaterial oder dgl. sowie einer Feuchtigkeitssperre (30),
**dadurch gekennzeichnet,**
daß die Feuchtigkeitssperre (30) vollständig in das Außenteil (12) und/oder in das Innenteil (14) eingebettet ist, wobei das Außenteil (12) aus kochfestem Baumwollmaterial, Zellstoffmaterial oder dgl. besteht und die Feuchtigkeitssperre (30) aus kochfester Kunststoffolie oder dgl. gebildet ist.

2. Windelhöschen nach Anspruch 1,
**dadurch gekennzeichnet,**
daß die als Feuchtigkeitssperre (30) vorgesehene Kunststoffolie oder dgl. in wenigstens einer Lage zwischen zwei Lagen (32, 34) aus Baumwollmaterial, Zellstoffmaterial oder dgl. des Außenteiles (12) und/oder des Innenteiles (14) angeordnet ist, wobei die eine Lage aus Kunststoffolie und die beiden Lagen (32, 34) aus Baumwollmaterial, Zellstoffmaterial oder dgl. des Außenteiles (12) bzw. des Innenteiles (14) zueinander deckungsgleich und miteinander unlösbar verbunden, vorzugsweise im randseitigen Bereich (36) vernäht sind.

3. Windelhöschen nach Anspruch 2,
**dadurch gekennzeichnet,**
daß die beiden Lagen (32, 34) aus einem, insbesondere grobmaschigem, Baumwollnetz oder dergleichen bestehen.

4. Windelhöschen nach Anspruch 2 oder 3,
**dadurch gekennzeichnet,**
daß weitere Lagen (40) aus Baumwollmaterial, Zellstoffmaterial oder dgl. zwischen den beiden Lagen (32, 34) aus Baumwollmaterial, Zellstoffmaterial oder dgl. des Außenteiles (12) bzw. des Innenteiles (14) eingebracht und mit diesen unlösbar verbunden sind.

5. Windelhöschen nach wenigstens einem der Ansprüche 2 bis 4,
**dadurch gekennzeichnet,**
daß mindestens eine Lage (42) aus Vliesstoff oder dgl. zwischen den beiden Lagen (32, 34) aus Baumwollmaterial, Zellstoffmaterial oder dgl. des Außenteiles (12) bzw. des Innenteiles (14) eingebracht und mit diesen unlösbar verbunden ist.

6. Windelhöschen nach wenigstens einem der Ansprüche 2 bis 5,
**dadurch gekennzeichnet,**
daß mindestens eine Lage (48) aus Watte oder dgl. zwischen den beiden Lagen (32, 34) aus Baumwollmaterial, Zellstoffmaterial oder dgl. des Außenteiles (12) bzw. des Innenteiles (14) eingebracht und mit diesen unlösbar verbunden ist.

7. Windelhöschen nach wenigstens einem der Ansprüche 2 bis 6,
**dadurch gekennzeichnet,**
daß mindestens eine Lage (50) aus Molton oder dgl. zwischen den beiden Lagen (32, 34) aus Baumwollmaterial, Zellstoffmaterial oder dgl. des Außenteiles (12) bzw. des Innenteiles (14) eingebracht und mit diesen unlösbar verbunden ist.

8. Windelhöschen nach wenigstens einem der Ansprüche 1 bis 7,
**dadurch gekennzeichnet,**
daß die als Feuchtigkeitssperre (30) vorgesehene Kunststoffolie säurebeständig ausgebildet ist.

9. Windelhöschen nach wenigstens einem der Ansprüche 1 bis 8,
**dadurch gekennzeichnet,**
daß das Innenteil (14) mit dem Außenteil (12) über eine lösbare Befestigungsvorrichtung, insbesondere einen Klettverschluß (20, 20', 20''), einen Druckknopf-, Rast-, Hak- oder dgl. -verschluß verbindbar ist.

10. Windelhöschen nach Anspruch 9,
**dadurch gekennzeichnet,**
daß der Klettverschluß (20) das Innenteil (14) mit dem Außenteil (12) im Bereich wenigstens einer der einander zugeordneten Endseiten (22, 24) von Außenteil (12) und Innenteil (14) verbindet.

11. Windelhöschen nach wenigstens einem der Ansprüche 1 bis 10,
**dadurch gekennzeichnet,**
daß das Außenteil (12) mittels Klettverschluß (16, 16', 16'') oder dgl. weitenverstellbar verschließbar ist.

12. Windelhöschen nach wenigstens einem der Ansprüche 1 bis 11,
**dadurch gekennzeichnet,**
daß der Klettverschluß (16, 16', 16''; 20, 20', 20'') von Außenteil (12) und/oder Innenteil (14) mit integriertem Fusselschutz (44; 46) oder dergleichen Schutzabdeckung versehen ist/sind.

13. Windelhöschen nach wenigstens einem der Ansprüche 1 bis 12,
**dadurch gekennzeichnet,**
daß das Außenteil (12) jeweils randseitig wenigstens im Bereich der Beine des Babys bzw. Kleinkindes mit einem Gummiband (18) oder dgl. versehen ist.
